# EUROPEAN PATENT APPLICATION

(11) **EP 4 480 410 A1**
(43) Date of publication of application: **25.12.2024**
(21) Application number: 23756512.2
(22) Date of filing: 05.01.2023
(51) Int. Cl.: A61B 5/1495, A61B 5/145, A61B 5/155, A61B 5/00

(54) **METHOD FOR COMPENSATING CALIBRATION FACTOR**

(30) Priority: 17.02.2022 KR 20220021018
(71) Applicant: i-Sens, Inc., Seoul 06646 (KR)
(72) Inventor: LEE, David, Seoul 06646 (KR); KANG, Young Jea, Seoul 06646 (KR)
(74) Representative: Maiwald GmbH
(86) International application number: PCT/KR2023/000253
(87) International publication number: WO 2023/158099

(57) **Abstract**

The present invention relates to a method for compensating a calibration factor in a system for measuring continuous biometric information and, more particularly, to a method for compensating a calibration factor, wherein: an offset is obtained in consideration of the sensor characteristics that change over time of sensor use after a sensor is inserted into the body, thereby accurately compensating a calibration factor in consideration of the sensor characteristics that change in real time; and a reference average value is obtained from biosignals from a first time point to a second time point after a sensor is inserted, and an offset is obtained from the difference between the reference average value and a comparison average value that changes over time of sensor use, thereby obtaining an offset in an accurate and noise-resistant manner.

## Description

### Technical Field

The present disclosure relates to a method of compensating a calibration factor in a system for continuously measuring biometric information, and more particularly, to a method of compensating a calibration factor, in which an offset is obtained in consideration of sensor characteristics changing over time during sensor use, after a sensor is inserted into the body, thereby accurately compensating a calibration factor in consideration of sensor characteristics changing in real time, and a reference average value is obtained from biosignals measured by the sensor from a first point in time to a second point in time after a sensor is inserted, and an offset is obtained from a difference between the reference average value and a comparison average value changing over time during sensor use, thereby obtaining an offset in an accurate and noise-resistant manner.

### Background Art

Diabetes is a chronic disease that occurs frequently in modern people, and in Korea, it affects 2 million or more people, 5% of the total population.

Diabetes occurs when the insulin produced by the pancreas is absolutely or relatively insufficient due to various causes such as obesity, stress, bad eating habits, congenital heredity and the like, and the blood sugar content in the blood increases absolutely due to the inability to calibrate a blood sugar balance.

Blood usually contains a certain concentration of glucose, and tissue cells obtain energy therefrom.

However, when glucose increases more than necessary, it is not stored properly in the liver, muscles, fat cells or the like, and accumulates in the blood, and as a result, diabetic patients maintain much higher blood sugar levels than normal people, and as the excessive blood sugar passes through the tissues as it is and is excreted in the urine, the sugar that is absolutely necessary for each tissue of the body becomes insufficient, causing abnormalities in each tissue of the body.

Diabetes is characterized by having almost no symptoms in the early stages, but as the disease progresses, it causes symptoms unique to diabetes, such as polydipsia, polyphagia, polyuria, weight loss, whole body fatigue, itchy skin, and long-lasting wounds on the hands and feet that may not heal, and as the disease progresses further, complications such as visual impairment, high blood pressure, kidney disease, stroke, periodontal disease, muscle cramps and neuralgia, gangrene and the like may appear.

To diagnose this type of diabetes and manage it so that it does not progress to complications, systematic blood sugar measurement and treatment should be performed in parallel.

Since diabetes requires constant blood sugar measurement for management, the demand for devices related to blood sugar measurement is steadily increasing. Various studies have confirmed that when diabetic patients strictly control their blood sugar, the occurrence of diabetic complications is significantly reduced. Accordingly, it is very important for diabetic patients to regularly measure their blood sugar to control their blood sugar.

For blood sugar management of diabetic patients, a blood glucose meter (finger prick method) is mainly used. Although this blood collection type glucose meter helps in blood sugar management of diabetic patients, it is difficult to accurately identify blood sugar levels changing frequently, because only the results at the time of measurement are displayed. In addition, blood collection type glucose meters require blood to be drawn every time to measure blood sugar levels several times a day, a significant burden for diabetic patients.

Diabetic patients generally go through hyperglycemia and hypoglycemia, and emergency situations occur in hypoglycemia. Hypoglycemia occurs when sugar levels do not last long, and may cause loss of consciousness or, in the worst case, death. Therefore, it is very important for diabetic patients to immediately detect hypoglycemia. However, blood collection type glucose meters measuring blood sugar levels intermittently have clear limitations.

To overcome the limitations of this blood collection type glucose meter, a continuous glucose monitoring system (CGMS) that is inserted into the body and measures blood sugar levels at intervals of several minutes has been developed, and this may be used to easily manage diabetic patients and respond to emergencies.

The continuous glucose monitoring system is configured to include a sensor transmitter that is attached to a user's body part and measures and transmits a biosignal indicating a blood glucose value from a body fluid, a communication terminal that converts the biosignal transmitted from the sensor transmitter into a blood glucose value and outputs it to the user, and the like. The sensor transmitter is equipped with a continuous blood glucose measurement sensor that is partially inserted into the human body, and the sensor is inserted into the human body for a certain period of use, for example, approximately 15 days, and continuously measures the biosignal. The sensor transmitter periodically measures the biosignal from the body fluid, and a blood glucose management application is installed in the communication terminal to periodically receive the biosignal from the sensor transmitter, calculate the blood glucose value from the received biosignal, and output the same to the user.

The sensor of the sensor transmitter is continuously inserted into the skin for a predetermined period of use, and the sensor sensitivity may vary depending on the body part into which the sensor is inserted or depending on the user wearing the sensor, and even if the sensor insertion location in the body part is the same, the sensor sensitivity changes over time. Therefore, the user's blood glucose value should be calibrated using a calibration factor for calibrating the measured biometric information according to the changing sensitivity.

To provide accurate blood glucose values to the user, the biosignals received from the sensor transmitter should be initially calibrated, and then continuously calibrated at regular calibration intervals during the sensor transmitter's usage period. In more detail, during the sensor transmitter's usage period, a calibration factor is obtained from a reference blood glucose value measured by a separate measuring device at each calibration interval and a biosignal value, for example, the current value, received from the sensor transmitter, and then the blood glucose value is obtained by applying the calibration factor to the biosignal value received from the sensor transmitter.

The calibration factor (F) may be obtained as the ratio of the reference blood glucose value (BG) and the current value (i) received from the sensor transmitter, as illustrated in the following mathematical expression 1. Calibration factor = reference blood glucose value/current value

The sensor's characteristics may change as the sensor's usage period elapses during the sensor's specified usage period. For example, in the case in which biomolecules are densely packed around the sensor inserted into the body and encapsulate the sensor, the sensor may generate a biosignal with a lower current value than the normal state.

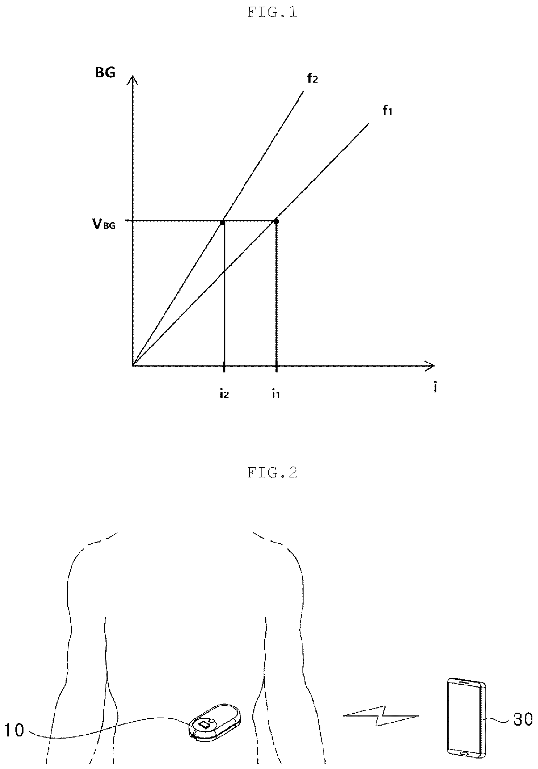

In this manner, when the sensor characteristics change, the current value measured by the sensor by corresponding to the reference blood glucose value (V_{BG}) of the same value decreases from i₁ to i₂ as illustrated in FIG. 1, and the calibration factor calculated therefrom is calculated as a relatively high value. In this manner, when the slope of the calibration factor is large, even if the value of the biosignal measured by the sensor changes slightly, the actual calibrated blood glucose value has a large difference, which may a problem of causing the calibrated blood glucose value to be inaccurate. To resolve this problem, the calibration factor may be compensated for with an offset when calculating the calibration factor.

In the related art, to compensate for the calibration factor, a preset offset is provided, based on the size of the calibration factor. For example, when the size of the calibration factor is a certain value or more, the calibration factor was compensated with the value of the offset O₁, and when the size of the calibration factor is a certain value or less, the calibration factor was compensated with the offset O₂. However, this compensation method of the calibration factor of the related art does not consider real-time changes in sensor characteristics, and furthermore, it has the problem of not accurately compensating the calibration factor because it does not consider sensor characteristics changing differently depending on the type of sensor, the part where the sensor is worn, the user using the sensor, or the time elapsing of use of the sensor.

### Summary of Invention

### Technical Problem

An aspect of the present disclosure is to provide a method of compensating a calibration factor, in which an offset is obtained by considering sensor characteristics changing over a usage time of a sensor after the sensor is inserted into the body, thereby accurately compensating the calibration factor by using the offset, to resolve problems of the offset-based calibration factor compensation method of the related art mentioned above.

Another aspect of the present disclosure is to provide a method of compensating a calibration factor, in which a reference average value is obtained from biosignals measured by a sensor from a first point in time to a second point in time after a sensor is inserted, and an offset is obtained from a difference between the reference average value and a comparison average value changing over time during sensor use, thereby obtaining an offset in an accurate and noise-resistant manner.

According to another aspect of the present disclosure is to provide a method of compensating a calibration factor, in which an accurate offset reference value may be obtained by varying a period for acquiring biometric information of a sensor after the sensor is inserted depending on whether the sensor is stabilized to calculate a reference average value.

### Solution to Problem

According to an aspect of the present disclosure, a method of compensating a calibration factor in a continuous biometric information measurement system includes receiving a biosignal from a sensor inserted into a body that measures biometric information of a user, obtaining a reference average value from the biosignal, obtaining an offset from a difference between a comparison average value up to a point in time at which a reference biometric value is input and the reference average value based on that the reference biometric value for calibrating the biosignal is input, and determining the calibration factor using the offset, and generating a calibrating biometric value by applying the biosignal to the calibration factor.

In detail, the obtaining the reference average value may include extracting biosignals received from a first point in time to a second point in time after the sensor is inserted into the body, and obtaining the reference average value from the first point in time to the second point in time based on the extracted biosignals.

The second point in time may be after a sensor stabilization time has elapsed based on the first point in time.

The stabilization determination time may be preset.

The obtaining the reference average value further may include determining whether the sensor is stabilized based on the biosignals at the second point in time, wherein the obtaining the reference average value may calculate the reference average value from the first point in time to the second point in time, based on the biosignals when the sensor is determined to be stabilized.

The obtaining the reference average value may further include determining whether the sensor is stabilized based on the biosignals received from the sensor after the second point in time at which the sensor is determined to not be stabilized, wherein the obtaining the reference average value may calculate the reference average value based on the biometric information from the first point in time to a point in time at which the sensor is stabilized, when the sensor is determined to be stabilized.

A stabilization determination time in another embodiment of the present disclosure may vary depending on whether the sensor is stabilized.

The obtaining the comparative average value may include extracting a biosignal received from a first point in time after the sensor is inserted into the body to a point in time at which the reference blood glucose value is input, and obtaining a comparative average value from the extracted biosignal.

Whether the sensor is stabilized may be determined based on at least one of a rate of change of the biosignal, a difference between the reference biometric value and the biosignal, a rate of change of sensor sensitivity, and a coefficient of variation.

The calibration factor may be determined through obtaining a sensor sensitivity from the reference biometric value and the biosignal, and applying the offset to the sensor sensitivity.

The biosignal may be a blood sugar signal of the user.

### Advantageous Effects of Invention

A method of compensating a calibration factor in a continuous biometric information measuring device according to the present disclosure has the following effects.

First, the method of compensating a calibration factor according to the present disclosure obtains an offset by considering sensor characteristics changing with the passage of time of use of a sensor after the sensor is inserted into the body, thereby accurately compensating a calibration factor by considering the sensor characteristics changing in real time according to the sensor type, user, body part into which the sensor is inserted, and the actual time elapsing of use of the sensor.

Second, a method of compensating a calibration factor according to the present disclosure obtains a reference average value from a biometric signal measured by a sensor from a first point in time to a second point in time after the sensor is inserted, and obtains an offset from a difference between the reference average value and a comparative average value changing with the passage of time of use of the sensor, thereby obtaining an offset in an accurate and noise-resistant manner.

Third, a method of compensating a calibration factor according to the present disclosure obtains an offset by varying a period for acquiring biometric information of a sensor after the sensor is inserted depending on whether the sensor is stabilized to calculate a reference average value, thereby obtaining an accurate offset reference value and accurately compensating a calibration factor.

### Brief Description of Drawings

FIG. 1 illustrates an example of a calibration factor calculated from a reference blood glucose value and a blood sugar signal.
FIG. 2 is a schematic diagram illustrating a measurement system for continuous biometric information according to an embodiment of the present disclosure.
FIG. 3 is a functional block diagram illustrating a compensation device for a calibration factor according to the present disclosure.
FIG. 4 is a functional block diagram illustrating an example of an offset generation unit according to the present disclosure.
FIG. 5 is a flowchart illustrating a method of compensating a calibration factor in a measurement system for continuous biometric information according to the present disclosure.
FIG. 6 is a flowchart illustrating an example of a method of obtaining an offset in the present disclosure.
FIG. 7 is a diagram illustrating an example of obtaining an offset in the present disclosure.
FIG. 8 is a flowchart illustrating an example of obtaining a reference average value.

### Best Mode for Invention

It should be noted that the technical terms used in the present disclosure are used only to describe specific embodiments and are not intended to limit the present disclosure. In addition, the technical terms used in the present disclosure should be interpreted as meanings generally understood by those skilled in the art to which the present disclosure belongs, unless specifically defined in the present disclosure to have different meanings, and should not be interpreted in an excessively comprehensive or excessively narrow sense. In addition, when the technical terms used in the present disclosure are incorrect technical terms that do not accurately express the idea of the present disclosure, they should be replaced with technical terms that may be correctly understood by those skilled in the art.

In addition, the singular expressions used in the present disclosure include plural expressions unless the context clearly indicates otherwise. In the present disclosure, the terms "configured", "include" and the like should not be interpreted as necessarily including all of the various components or various steps described in the present disclosure, and should be interpreted to mean that some of the components or some steps may not be included, or that additional components or steps may be further included.

In addition, it should be noted that the attached drawings are only intended to facilitate easy understanding of the spirit of the present disclosure, and should not be construed as limiting the spirit of the present disclosure by the attached drawings.

Hereinafter, with reference to the attached drawings, a method of compensating a calibration factor in a continuous biometric information measurement system according to the present disclosure will be examined in more detail.

FIG. 2 is a schematic diagram illustrating a continuous biometric information measurement system according to an embodiment of the present disclosure.

Hereinafter, blood glucose values are described as an example of biometric information and reference blood glucose values are described as an example of reference biometric information, but various biometric information other than blood sugar values may be measured depending on the field to which the present disclosure is applied.

Referring to FIG. 2, a continuous biometric information measurement system according to an embodiment of the present disclosure includes a sensor transmitter 10 and a communication terminal 30.

The sensor transmitter 10 is attached to the body, and when the sensor transmitter 10 is attached to the body, one end of a sensor of the sensor transmitter 10 is inserted into the skin to periodically measure a biosignal indicating a blood glucose value, for example, a blood sugar signal, from body fluid of the human body.

The communication terminal 30 is a terminal that receives a blood sugar signal from the sensor transmitter 10, calibrates the received blood sugar signal with a calibration factor to be converted into the unit of calibrated blood glucose value, and then displays the same to the user. A terminal that may communicate with the sensor transmitter 10, such as a smart phone, a tablet PC, a laptop or the like, may be used. Of course, the communication terminal 30 is not limited thereto, and any type of terminal which includes a communication function and in which a program or application may be installed may be used.

For example, the sensor transmitter 10 generates a blood sugar signal, for example, a current signal, representing the user's blood glucose value, and transmits the blood sugar signal to the communication terminal 30, and the communication terminal 30 calibrates the blood sugar signal with a calibration factor to be converted into the unit of calibrated blood glucose value. Depending on the field to which the present disclosure is applied, the sensor transmitter 10 may directly convert the blood sugar signal into a unit of blood glucose value, and the communication terminal 30 may calibrate the blood glucose value received from the sensor transmitter 10 with the calibration factor. According to the field to which the present disclosure is applied, the blood sugar signal is calibrated by the calibration factor in the sensor transmitter 10 and converted into a unit of the calibrated blood glucose value, and the communication terminal 30 may receive the calibrated blood glucose value and output the same to the user.

The sensor transmitter 10 transmits the blood sugar signal to the communication terminal 30 periodically or in real time at the request of the communication terminal 30 or at set times. For data communication between the sensor transmitter 10 and the communication terminal 30, the sensor transmitter 10 and the communication terminal 30 may be connected to each other by wired communication using a USB cable or the like, or may be connected by wireless communication using infrared communication, NFC communication, Bluetooth, or the like.

When communication is connected between the sensor transmitter 10 and the communication terminal 30, the initial calibration factor is calculated using the reference blood glucose value measured by a separate blood sugar meter (not illustrated) after the initial stabilization of the sensor transmitter 10, and the initial calibration of the blood sugar signal is performed using the initial calibration factor. Thereafter, the communication terminal 30 calibrates the blood sugar signal received from the sensor transmitter 10 using the initial calibration factor and outputs and provides the calibrated blood glucose value to the user.

In this case, the initial stabilization is determined as initial stabilization after a certain period of time, for example, 2 hours, after inserting the sensor into the body, or the initial stabilization may be determined based on the blood sugar signal. However, the initial stabilization is the minimum stabilization time or condition required to inform the user of the calibrated blood glucose value as soon as possible after inserting the sensor into the body, and additional time, for example, about 3 to 10 days, may be required until the actual sensor is stabilized.

To accurately calibrate the blood sugar signal in the sensor transmitter 10 after the initial stabilization, the communication terminal 30 calculates a new calibration factor using the reference blood glucose value measured by a separate blood sugar meter at each correction cycle during the use period of the sensor transmitter 10, and calculates the calibrated blood glucose value by calibrating the blood sugar signal received from the sensor transmitter using the new calibration factor, and outputs the calculated calibrated blood glucose value to the user.

In this case, when calculating the initial calibration factor or the new calibration factor, an offset that varies depending on the sensor characteristics is calculated, and the calibration factor may be compensated for with the calculated offset. In this manner, by considering the sensor characteristics changing in real time when calculating the offset used to compensate for the calibration factor, compared to the compensation method of the related art that uses a preset offset, the offset may be calculated by considering the sensor characteristics changing depending on the type of sensor, the user using the sensor, the body part into which the sensor is inserted, and the time over which the sensor is inserted and used in the body, and the calibration factor may be compensated more accurately therefrom.

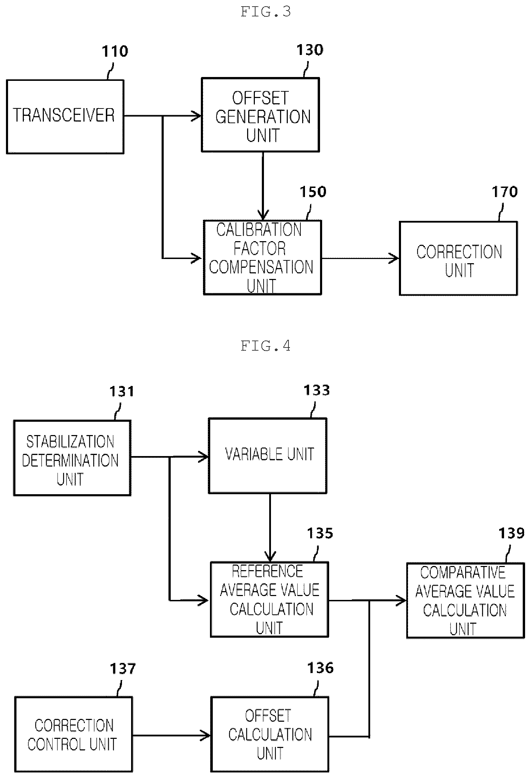

FIG. 3 is a functional block diagram illustrating the calibration factor compensation device according to the present disclosure.

In the present disclosure, if the calculation and compensation of the calibration factor are performed in the communication terminal, the corresponding calibration factor compensation device may be implemented in the communication terminal, but if the calculation and compensation of the calibration factor are performed in the sensor transmitter, the calibration factor compensation device may be implemented in the sensor transmitter.

Looking more specifically with reference to FIG. 3, a transceiver 110 receives the blood sugar signal measured by the sensor transmitter. In this case, the blood sugar signal may be a current value measured from a body fluid by a sensor inserted into the body.

An offset generation unit 130 obtains a reference average value from the blood sugar signal from the first point in time to the second point in time after the sensor is inserted, and when a correction cycle arrives thereafter, obtaines an offset from a difference between the comparison average value from the first point in time to the correction cycle and the reference average value. In more detail, the blood sugar signal measured by the sensor from the first point in time to the second point in time after the sensor is inserted is acquired, and the reference average value is calculated from the blood sugar signal from the first point in time to the second point in time. The offset generation unit 130 obtains an offset considering the changed sensor characteristics from the difference between the comparative average value calculated from the blood sugar signal measured by the sensor from the first point in time to the calibration point in time and the reference average value.

A calibration factor compensation unit 150 obtains a calibration factor from the ratio of the blood sugar signal received through the transceiver 110 and the reference blood glucose value when the calibration cycle arrives or when a reference blood glucose value for calibrating the blood sugar signal is input regardless of the calibration cycle, and compensates for the calibration factor by applying the offset calculated at the calibration point in time to the calibration factor.

A correction unit 170 converts the blood sugar signal into a unit using the compensated calibration factor when receiving a blood sugar signal measured by the sensor after the calibration point in time and generates a calibration ed blood glucose value.

FIG. 4 is a functional block diagram illustrating an example of an offset generation unit according to the present disclosure.

Referring to FIG. 4 in more detail, a reference average value calculation unit 135 obtains a reference average value from the blood sugar signal from the first point in time to the second point in time, measured by the sensor. The reference average value is a reference value used to obtain an offset for compensating a calibration factor during the usage period of the sensor, and as the reference average value, a blood sugar signal acquired from the first point in time to the second point in time, for example, an average value, a median value, an arithmetic mean value, a geometric mean value, and the like of the current values measured by the sensor from the first point in time to the second point in time, may be used.

Preferably, a stabilization determination unit 131 determines whether the sensor is stabilized when a preset second point in time has elapsed after the sensor is inserted, and based on the determination result of the stabilization determination unit 131, the reference average value calculation unit 135 may obtains the reference average value from the blood sugar signal measured by the sensor from the first point in time to the second point in time in the case in which the sensor is stabilized at the second point in time.

However, based on the determination result of the stabilization determination unit 131, in the case in which the sensor is not stabilized at the second point in time, a variable unit 133 extends and varies the stabilization determination time from the second point in time, and the stabilization determination unit 131 determines whether the sensor is stabilized at the varied stabilization determination time. Depending on the field to which the present disclosure is applied, the variable unit 133 may continuously extend the stabilization determination time to a set period based on the determination result of the stabilization determination unit 131 to determine whether the sensor is stabilized. In the case in which the sensor is stabilized at the varied stabilization determination time, the reference average value calculation unit 135 may obtain the reference average value from the blood sugar signal measured by the sensor from the first point in time to the varied stabilization determination time.

A comparative average value calculation unit 136 obtaines the comparative average value from the blood sugar signal measured by the sensor from the first point in time to the calibration point in time of the correction cycle or the calibration point in time that the user wants to correct regardless of the correction cycle. In this case, the comparative average value may be the average value, median value, arithmetic mean value, geometric mean value, or the like of the current values measured by the sensor from the first point in time to the calibration point in time.

Preferably, a correction control unit 137 determines whether the calibration point in time is valid based on the blood sugar signal of the sensor measured at the calibration point in time or the reference blood glucose value. For example, when the sensor is temporarily unstable or the user's blood glucose value is unstable, such as when the change rate of the blood sugar signal is the critical change rate or higher at the calibration point in time, or when the calibrated blood glucose value generated at the calibration point in time is out of the critical range, the calibration point in time may be delayed. The comparative average value calculation unit 136 obtaines the comparison average value at the time of calibration when the calibration time is determined to be valid, based on the determination result of the calibration control unit on whether the calibration time is valid.

An offset calculation unit 139 obtains the offset at the time of calibration from the difference between the reference average value and the comparison average value.

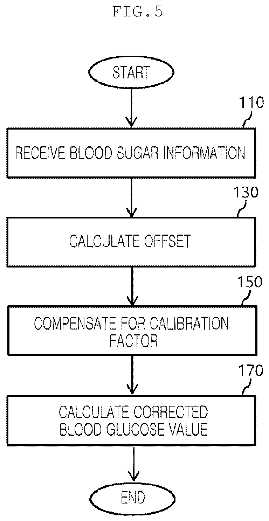

FIG. 5 is a flow chart illustrating a method of compensating a calibration factor in a continuous biometric information measurement system according to the present disclosure.

Looking in more detail with reference to FIG. 5, a blood sugar signal measured by a sensor is received (S110).

A reference average value is calculated from the blood sugar signal received from the first point in time to the sensor stabilization point in time after sensor insertion, and when the calibration point in time arrives thereafter, a comparison average value is calculated from the blood sugar signal received from the first point in time to the calibration point in time, and an offset is calculated from the difference between the reference average value and the comparison average value (S130).

At the calibration point in time, a calibration factor is calculated from the blood sugar signal measured by the sensor by corresponding to the reference blood glucose value measured using a separate blood sugar meter and the time at which the reference blood glucose value is measured, and the calculated calibration factor is compensated for as an offset (S150).

Preferably, the compensated calibration factor Fc may be calculated as in the following mathematical expression (2) . Fc=reference blood glucose value/(blood sugar signal-offset)

When a blood sugar signal is received from a sensor after the calibration point in time, the blood sugar signal is converted into a unit of a calibrateded blood glucose value using the compensated calibration factor, thereby performing calibration (S170). The calibrateded blood glucose value may be provided to the user through a communication terminal.

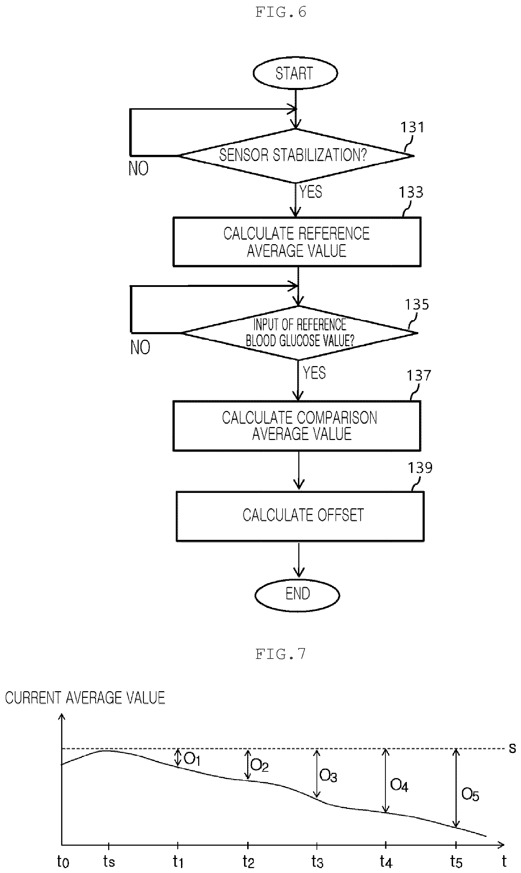

FIG. 6 is a flowchart illustrating an example of a method of obtaining an offset in the present disclosure.

Referring to FIG. 6 in more detail, it is determined whether the sensor is stabilized after the sensor is inserted (S131). In this case, the determination of whether the sensor is stabilized may be made when a preset stabilization determination time has arrived, but the stabilization determination time may vary depending on the field to which the present disclosure is applied.

In the case in which the sensor is stabilized, a reference average value is calculated from the blood sugar signal measured from the first point in time after the sensor is inserted to the stabilization determination point in time, for example, the second point in time (S133). In this case, the first point in time may be the same as the sensor insertion point in time, but the first point in time may be set to a point in time after a certain period of time has passed after the sensor is inserted. For example, the period between the first point in time and the second point in time may be set in various manners, such as 5 days from the time of sensor insertion, 1 day after sensor insertion being the first point in time and 5 days after sensor insertion being the second point in time, 3 days after sensor insertion being the first point in time and 5 days after sensor insertion being the second point in time, or the like.

When a user command to input a reference blood glucose value is input at a calibration point in time according to a calibration cycle or a calibration point in time unrelated to a calibration cycle (S135), a comparison average value is obtained from the blood sugar signals measured from the first point in time to the calibration point in time (S137).

An offset is obtained from the difference between the reference average value and the comparison average value (S139).

FIG. 7 is a drawing illustrating an example of obtaining an offset in the present disclosure.

As illustrated in FIG. 7, the average value of the blood sugar signal, for example, the current value, measured by the sensor from the first point in time t₀ to the point in time tₛ at which the sensor is stabilized is calculated.

The offset used to compensate for the calibration factor at the first calibration point in time t₁ is calculated as the difference O₁ between the reference average value (s) and the comparison average value at the first calibration point in time, for example, between the average values of the current values up to the first calibration point in time. For example, when the difference between the reference average value and the comparison average value is -0.2, -0.2 is used as the offset for the first calibration point in time to compensate for the calibration factor.

Similarly, the offset is obtained from the difference between the reference average value and the comparison average value at the second calibration point in time t₂, the third calibration point in time t₃, the fourth calibration point in time t₄, and the fifth calibration point in time t₅ to compensate for the calibration factor at the second calibration point in time t₂, the third calibration point in time t₃, the fourth calibration point in time t₄, and the fifth calibration point in time t₅.

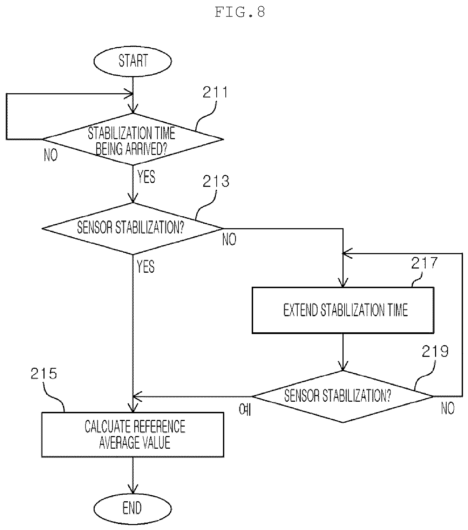

FIG. 8 is a flow chart illustrating an example of obtaining the reference average value.

Referring to FIG. 8 in more detail, it is determined whether the stabilization determination time has arrived after the sensor is inserted (S211). In this case, the stabilization determination point in time may be set in various manners depending on the sensor characteristics.

In the case in which the stabilization determination time has arrived, it is determined whether the sensor has stabilized (S213). In this case, whether the sensor is stabilized may be determined based on the trend of the blood sugar signal measured by the sensor (for example, rate of change, deviation, or the like), the deviation of the blood sugar signal from the reference blood glucose value, the change in sensitivity, the coefficient of variation, or the like.

In the case in which the sensor is determined to be stabilized, the reference average value is obtained from the blood sugar signal measured by the sensor from the first point in time after sensor insertion to the stabilization determination time that has arrived (S215).

However, if the sensor is determined not to be stabilized until the stabilization determination time, the stabilization determination time is extended (S217). Whether the sensor is stabilized is determined again during the extended stabilization determination time, and if the sensor is determined to be stabilized during the extended stabilization determination time, the reference average value is obtained from the blood sugar signal measured by the sensor from the first point in time after sensor insertion to the extended stabilization determination time (S215).

However, if the sensor is determined not to be stabilized during the extended stabilization determination time, the stabilization determination time is extended again to determine whether the sensor is stabilized. In this manner, the stabilization determination time is continuously extended to a preset point in time based on whether the sensor is stabilized, and the sensor is determined to be stabilized. If the sensor is not stabilized until the preset point in time has passed, the calibrated blood glucose value may be calculated without compensating the calibration factor calculated at the calibration point in time. In another embodiment of the present disclosure, if the sensor is not stabilized until the preset point in time has passed, the user may be notified to replace the sensor.

Meanwhile, the embodiments of the present disclosure described above may be written as a program that may be executed on a computer, and may be implemented in a general-purpose digital computer that operates the program using a computer-readable recording medium.

The computer-readable recording medium includes a storage medium such as a magnetic storage medium (for example, a ROM, a floppy disk, a hard disk, etc.), an optical reading medium (for example, CD-ROM, DVD, etc.), and a carrier wave (for example, transmission via the Internet).

The present disclosure has been described with reference to the embodiments illustrated in the drawings, but these are merely illustrative, and those skilled in the art will understand that various modifications and equivalent other embodiments are possible therefrom. Therefore, the true technical protection scope of the present disclosure should be determined by the technical idea of the attached claims.

## Claims

1. A method of compensating a calibration factor, comprising:
receiving a biosignal from a sensor inserted into a body that measures biometric information of a user (S110);
obtaining a reference average value from the biosignal (S133);
obtaining an offset from a difference between a comparison average value up to a point in time at which a reference biometric value is input and the reference average value based on that the reference biometric value for calibrating the biosignal is input(S130); and
determining the calibration factor using the offset (S150), and generating a calibrated biometric value by applying the biosignal to the calibration factor (S170).

2. The method of compensating for a calibration factor of claim 1, wherein the obtaining the reference average value (S133) includes,
extracting biosignals received from a first point in time to a second point in time after the sensor is inserted into the body; and
obtaining the reference average value from the first point in time to the second point in time based on the extracted biosignals.

3. The method of compensating for a calibration factor of claim 2, wherein the second point in time (t₂) is after a sensor stabilization time (tₛ) has elapsed based on the first point in time (t₁).

4. The method of compensating for a calibration factor of claim 3, wherein the sensor stabilization time (tₛ) is preset.

5. The method of compensating for a calibration factor of claim 4, wherein the obtaining the reference average value (S133) further includes:
determining whether the sensor is stabilized based on the biosignals at the second point in time,
wherein the obtaining the reference average value calculates the reference average value from the first point in time to the second point in time based on the biosignals when the sensor is determined to be stabilized.

6. The method of compensating for a calibration factor of claim 5, wherein the obtaining the reference average value (S133) further includes:
determining whether the sensor is stabilized based on the biosignals received from the sensor after the second point in time at which the sensor is determined to not be stabilized,
wherein the obtaining the reference average value calculates the reference average value based on the biometric information from the first point in time to a point in time at which the sensor is stabilized, when the sensor is determined to be stabilized (S131).

7. The method of compensating for a calibration factor of claim 3, wherein the sensor stabilization time varies depending on whether the sensor is stabilized.

8. The method of compensating for a calibration factor of claim 1, wherein the obtaining the comparative average value includes:
extracting biosignals received from a first point in time after the sensor is inserted into the body to a point in time at which a reference blood glucose value is input; and
obtaining a comparative average value from the extracted biosignals.

9. The method of compensating for a calibration factor of any one of claims 5 to 8, wherein, whether the sensor is stabilized is determined based on at least one of a rate of change of the biosignal, a difference between the reference biometric value and the biosignal, a rate of change of sensor sensitivity, and a coefficient of variation.

10. The method of compensating for a calibration factor of claim 1, wherein the calibration factor is determined through,
obtaining a sensor sensitivity from the reference biometric value and the biosignal; and
applying the offset to the sensor sensitivity.

11. The method of compensating for a calibration factor of claim 1, wherein the biosignal is a blood sugar signal of the user.
